# EUROPEAN PATENT SPECIFICATION

(11) **EP 3 414 566 B1**
(45) Date of publication and mention of the grant of the patent: **30.12.2020**
(21) Application number: 17706106.6
(22) Date of filing: 10.02.2017
(51) Int. Cl.: G01N 33/28, G01V 9/00

(54) **THERMAL MATURITY DETERMINATION OF ROCK FORMATIONS USING MUD GAS ISOTOPE LOGGING**
BESTIMMUNG DER THERMISCHEN REIFUNG VON FELSFORMATIONEN MITTELS SCHLAMMGASISOTOPENMESSUNG
DÉTERMINATION DE MATURITÉ THERMIQUE DE FORMATIONS ROCHEUSES PAR DIAGRAPHIE D'ISOTOPES DE GAZ DE BOUES

(30) Priority: 10.02.2016 US 201662293498 P
(43) Date of publication of application: 19.12.2018
(73) Proprietor: Saudi Arabian Oil Company, Dhahran 31311 (SA)
(72) Inventor: HAKAMI, Ahmed, Dhahran 31311 (SA); ELLIS, Leroy, Dhahran 31311 (SA); ABDELBAGI, Sami, Dhahran 31311 (SA)
(74) Representative: Williams, Michael David
(86) International application number: PCT/US2017/017306
(87) International publication number: WO 2017/139532

(56) References cited:
- US-A1- 2005 256 646
- Owen A. Sherwood ET AL: "Hydrocarbon Maturity and Migration Analysis Using Production Gas Stable Isotopic Signatures in the Wattenberg Field, Denver Basin, Colorado, USA", Unconventional Resources Technology Conference, Denver, Colorado, 12-14 August 2013, 14 August 2013 (2013-08-14), pages 1989-1995, XP055587350, DOI: 10.1190/urtec2013-204

## Description

### BACKGROUND

### Field of the Disclosure

The disclosure generally relates to the drilling and extraction of oil, natural gas, and other resources, and more particularly to the evaluation of geological formations and identification of resources in such geological formations.

### Description of the Related Art

Oil, gas, and other natural resources are used for numerous energy and material purposes. The search and extraction of oil, natural gas, and other subterranean resources from the earth may cost significant amounts of time and money. The difficulty and costs associated with the extraction of some resources, such as shale gas, may increase the challenged in evaluating potential drilling locations such as basins and identifying the "sweet spots" in such locations that enable economically viable production of such resources. Existing techniques may be unable to accurately distinguish between different rock formations in some locations, thus limiting information available for selection of drilling sites and subsequent production capability. Some techniques, such as core sampling, may be inefficient and may result in significant increases in development time and costs. US2005256646 describes a method of interpreting mud gas samples in a drilling operation of a target area. Owen A. Sherwood ET AL: "Hydrocarbon Maturity and Migration Analysis Using Production Gas Stable Isotopic Signatures in the Wattenberg Field, Denver Basin, Colorado, USA", Unconventional Resources Technology Conference, Denver, Colorado 12-14 August, 14 August 2013 (2013-08-14), pages 1989-1995, describes the use of gas stable isotope signature as a hydrocarbon phase prediction and migration analysis tool.

### SUMMARY

Well logging techniques may provide for the evaluation of rock formations, including rock formations in basins or other features potentially having extractable shale gas. One or more exploratory wells may be drilling in a geographic feature, and well logging may be performed in each well to evaluate differences in rock formations and potential drilling sites. Such well logging may include core logging, mud logging, and mud gas isotope logging (MGIL).

MGIL involves direct isotopic and compositional measurements of formation gases sampled from the circulating mud stream during drilling. MGIL may provide measurements of carbon and hydrogen isotope data in various gases such as methane, propane, ethane, and so on.

One estimation performed by geochemical tools is the determination of the thermal maturity of rock formations. In conventional systems, the thermal maturity of a rock formation is extremely difficult to estimate once hydrocarbons are expelled from the source rock and begin to migrate along porous conduits that can range from tens of meters to hundreds of kilometers in length, thus irrevocably altering the chemical signature of the hydrocarbons. These changes are the result of many different processes, such as mixing with secondary contributors, gravity and density segregation, and distillation. In some instances, hydrocarbon generation kinetics may be derived using laboratory pyrolysis of source rock samples to model subsurface conditions and quantity resultant hydrocarbon products that are generated; however, such techniques require representative source rock samples that are difficult to obtain and may instead may use poor quality analogue samples. Moreover, conventional systems are difficult to check the model accuracy against field data from the source rock.

As explained herein, the use of MGIL with shale formations may result in the correlation of in situ thermal maturity of rock formations with the compositional and isotopic products of associated hydrocarbon generation, thus making the samples accurately representative and self-sourcing.

In one embodiment, a method for determining the thermal maturity of a rock formation in a geological region of interest for hydrocarbon production is provided. The method includes drilling a plurality of exploration wells to access the rock formation and obtaining mud gas isotope logging data for each of the plurality of exploration wells. The mud gas isotope logging data includes isotopic values associated with an isotope in a mud gas. The method also includes obtaining a vitrinite reflectance equivalent for each of the plurality of exploration wells and determining a relationship between vitrinite reflectance equivalent and the isotopic values, the vitrinite reflectance equivalent indicating a thermal maturity of the rock formation.

In some embodiments, the method includes determining the vitrinite reflectance equivalent using the relationship and isotopic values associated with an additional exploration well accessing a second rock formation and identifying a thermal maturity of the second rock formation using the vitrinite reflectance equivalent. In some embodiments, the method includes identifying a drilling location in the geological region of interest for hydrocarbon production using the thermal maturity indicated by the vitrinite reflectance equivalent. In some embodiments, the mud gas comprises methane and the isotope comprises carbon-13. In some embodiments, the mud gas comprises hydrogen and the isotope comprises deuterium. In accordance with the invention , the obtaining the vitrinite reflectance equivalent for each of the plurality of exploration wells includes determining, for each of the plurality of exploration wells, a temperature at which the maximum rate of hydrocarbon generation occurs in a kerogen sample from the respective exploration well during a pyrolysis analysis and calculating the vitrinite reflectance equivalent from the temperature for each of the plurality of exploration wells, &/or obtaining the vitrinite reflectance equivalent for each of the plurality of exploration wells includes determining an amount of bitumen in a core sample obtained from the respective exploration well and calculating the vitrinite reflectance equivalent from the bitumen amount for each of the plurality of exploration wells. In some embodiments, determining a relationship between vitrinite reflectance equivalent and the isotopic values includes performing a least squares regression analysis on a set of vitrinite reflectance equivalent values and a set of isotopic values.

In another embodiment, a system for determining the thermal maturity of a rock formation in a geological region of interest for hydrocarbon production is provided. The system includes mud gas isotope logging system configured to receive mud gas and determine isotopic values of associated with an isotope in the mud gas and a thermal maturity determination processing system having a processor and a non-transitory computer-readable memory accessible by the processor, the memory having executable code stored thereon. The executable code comprising a set of instructions that causes the processor to perform operations that include receiving, from the mud gas isotope logging system, mud gas isotope logging data for each of a plurality of exploration wells, the mud gas isotope logging data comprising isotopic values associated with the isotope in the mud gas and obtaining a vitrinite reflectance equivalent for each of the plurality of exploration wells, the vitrinite reflectance equivalent determine from a core sample from each of the plurality of exploration wells. The operations also include determining a relationship between vitrinite reflectance equivalent and the isotopic value, the vitrinite reflectance equivalent indicating a thermal maturity of the rock formation.

In some embodiments, the operations further include determining a vitrinite reflectance equivalent associated with an additional exploration well using the relationship and an isotopic value associated with the additional exploration well and identifying a thermal maturity of the rock formation using the vitrinite reflectance equivalent. In some embodiments, the mud gas comprises methane and the isotope comprises carbon-13. In some embodiments, the mud gas comprises hydrogen and the isotope comprises deuterium. In accordance with the invention , obtaining a vitrinite reflectance equivalent for each of the plurality of exploration wells includes determining, for each of the plurality of exploration wells, a temperature at which the maximum rate of hydrocarbon generation occurs in a kerogen sample from the respective exploration well during a pyrolysis analysis and calculating the vitrinite reflectance equivalent from the temperature for each of the plurality of exploration wells, &/or obtaining a vitrinite reflectance equivalent for each of the plurality of exploration wells includes determining an amount of bitumen in a core sample obtained from the respective exploration well and calculating the vitrinite reflectance from the bitumen amount for each of the plurality of exploration wells. In some embodiments, determining a relationship between vitrinite reflectance equivalent and the isotopic values comprises performing a least squares regression analysis on a set of vitrinite reflectance equivalent values and a set of isotopic values.

### BRIEF DESCRIPTION OF THE DRAWINGS

FIG. 1 depicts measurement-while-drilling gamma ray (GR) and density logs of stratigraphic cross-sections for the five exploratory wells drilled in a geographic region of interest in accordance with embodiments of the disclosure;
FIGS. 2A-2C are graphs of the distribution of total organic carbon (TOC) for three source rock formations in a geographic region of interest in accordance with embodiments of the disclosure;
FIG. 3 is a plot of the vitrinite reflectance equivalent vs. depth for a first source rock formation in accordance with embodiments of the disclosure;
FIG. 4 is a plot of the vitrinite reflectance equivalent vs. depth for a second source rock formation in accordance with embodiments of the disclosure;
FIGS. 5A-5E are images of representative core samples and tables of bitumen reflectance data obtained from a source rock formation for five exploratory wells in a geographic region of interest in accordance with embodiments of the disclosure;
FIG. 6 is a side-by-side depth plot of geological data for two exploration wells in a geological region of interest in accordance with embodiments of the disclosure;
FIG. 7 is a plot of methane carbon and deuterium isotopic data and vitrinite reflectance for a source rock formation in a geological region of interest in accordance with embodiments of the disclosure;
FIG. 8 is a block diagram of a process for determining thermal maturity from gas isotopic values obtained from mud gas isotope logging (MGIL) in accordance with embodiments of the disclosure; and
FIG. 9 is a block diagram of a well site having a mud gas isotope logging system and a thermal maturity determination system in accordance with an embodiment of the disclosure.

### DETAILED DESCRIPTION

The present disclosure will now be described more fully hereinafter with reference to the accompanying drawings, which illustrate embodiments of the disclosure. This disclosure may, however, be embodied in many different forms and should not be construed as limited to the illustrated embodiments set forth herein. Rather, these embodiments are provided so that this disclosure will be thorough and complete, and will fully convey the scope of the disclosure to those skilled in the art.

Embodiments of the disclosure include a method and a system for determining the thermal maturity of a rock (e.g., shale) formation having hydrocarbon (e.g., shale gas) resources. In some embodiments, two or more exploration wells may be drilled in a geographic region of interest. Mud gas isotope logging (MGIL) may be performed on the two or more exploration wells, and isotopic values in mud gas may be determined. As used herein, the term "isotopic values" refers to a value corresponding to or indicative of the amount of an isotope. The isotopic value may refer to an "isotopic signature" as known in the art and as calculated with respect to a Pee Dee Belemnite (PDB) standard. For example, the isotopic values of various carbon and hydrogen isotopes in mud gases such as methane (C1), ethane (C2), propane (C3), butane (C4), and pentane (C5) may be determined. The vitrinite reflectance equivalent may be determined for rock formations in the exploration wells from core samplings using known techniques. In accordance with the invention, , the temperature at which the maximum rate of hydrocarbon generation during pyrolysis analysis occurs in a kerogen sample obtained from the well may be determined, and the vitrinite reflectance equivalent may be determined from this maximum temperature. In accordance with the invention, the vitrinite reflectance equivalent may be additionally or alternatively calculated from solid bitumen present in core samples. Next, relationship between vitrinite reflectance equivalent, as an indicator of thermal maturity, and the isotopic values of a selected isotope analyzed via the MGIL may be determined. The thermal maturity of rock formations may then be determined from isotopic values from MGIL in additional exploration wells without performing core sampling or other costly or time-consuming techniques on such wells. Advantageously, the MGIL and subsequent thermal maturity determination may be performed while drilling such exploration wells to avoid other techniques that may delay drilling and increase drilling time.

### EXAMPLES

The following examples are included to demonstrate embodiments of the disclosure. It should be appreciated by those of skill in the art that the techniques and compositions disclosed in the example which follows represents techniques and compositions discovered to function well in the practice of the disclosure, and thus can be considered to constitute modes for its practice. However, those of skill in the art should, in light of the present disclosure, appreciate that many changes can be made in the specific embodiments which are disclosed and still obtain a like or a similar result without departing from the spirit and scope of the disclosure

Five wells (referred to as Well-A, Well-B, Well-C, Well-D, and Well-E) were drilled in a basin suspected as a source of shale gas and having middle-to-upper Jurassic organic-rich carbonate mudrocks. The wells were used to evaluate three potential source rock targets at different depths and referred to as Tuwaiq, Hanifa, and Jubaila. The wells were drilled vertically, cored, logged, and sampled to obtain the data discussed herein. The coring operations were performed to obtain a sufficient number of core samples to enable detailed source rock and maturity characterization across the three source rock targets. As explained below, mud gas isotope logging (MGIL) and source rock data were used to assess the thermal maturity of hydrocarbon shows, and both datasets were used to determine an accurate thermal maturity for thermal maturity estimation.

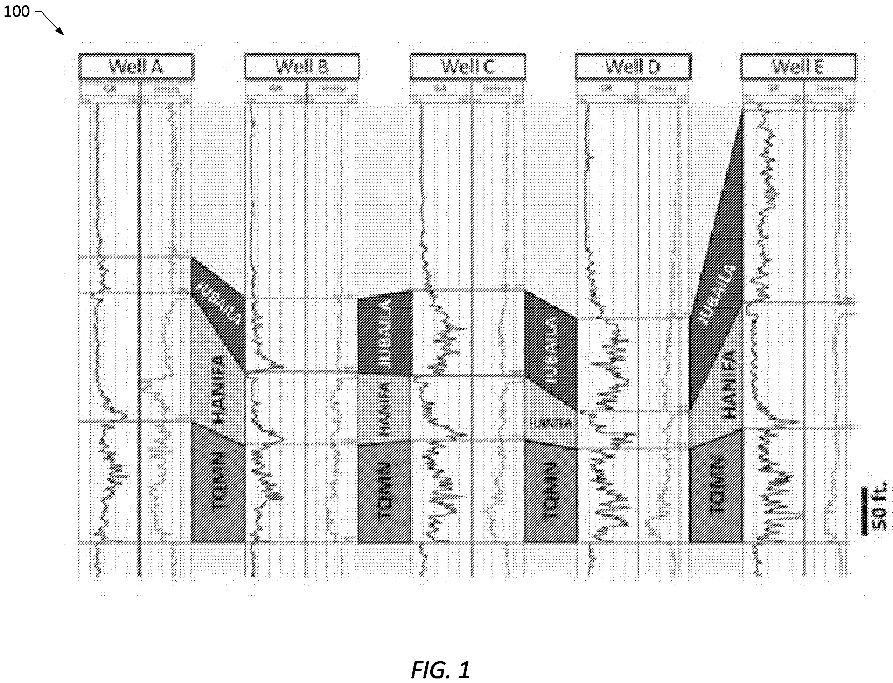

FIG. 1 depicts measurement-while-drilling gamma ray (GR) and density logs 100 of stratigraphic cross-sections for the five wells Well-A, Well-B, Well-C, Well-D, and Well-E. The logs 100 in FIG. 1 illustrate the lateral correlation of individual lithofacies within the middle and upper Jurassic source rock intervals. The laminated, organic-rich lime mudstone source rocks are relatively thick (up to 400 feet (ft)) and extend over large geographical areas. As explained below, the acquired source rock indicated that the Jubaila formation in the basin possesses good source rock quality while the Hanifa formation in the basin possesses poor litho-organic facies.

A data acquisition program was performed to acquire data on the three source rock targets. Core analysis was performed to capture full formation-thick cores across the source rocks to enable detailed characterization. As noted above, mud logs and MGIL were also performed to assess the type, origin, and thermal maturity of hydrocarbon shows.

The core intervals were selected by seismic data and correlation with historical offset wells. During drilling of the five wells, coring points were selected using near-bit measurement-while-drilling gamma ray (MWD GR) tools to identify formation tops. The core interval selection also enabled the capture of portions of the overlying and underlying formations to accurately select the transitions across formations while eliminating the risk of missing the targeted source rock intervals. The short cored intervals also provided data for future sedimentological work to enable analysis of the depositional history and engineering information on fracture growth behavior. RockEval® pyrolysis and LECO® were performed to identify source rock intervals and evaluate their quality (generative potential) and associated thermal maturity. The source rock maturity was also determined in some wells by direct organic petrographic analysis measuring the vitrinite reflectance (%%Ro) and other organic matter maceral-type reflectance.

The core analysis was performed on about seven hundred core samples collected from the five exploration wells: Well-A, Well-B, Well-C, Well-D, and Well-E. Table 1 summarizes the average geochemical properties for the three source rock formations Tuwaiq, Hanifa, and Jubaila explored by the five exploration wells. The average geochemical properties include total organic carbon (TOC) content, S1 pyrolysis yield (the S1 peak in a pyrolysis analysis such as RockEval®), S2 pyrolysis yield (the S2 peak in a pyrolysis analysis such as RockEval®), hydrogen index, and the temperature (Tmax) at which the maximum rate of hydrocarbon generation occurred in a kerogen sample during a pyrolysis analysis:

**Table 1: Average Geochemical Properties in Source Rock Formations**

| **Geochemical Attributes** | | **Source Rock Formation** | | |
|---|---|---|---|---|
| | | **Jubaila** | **Hanifa** | **Tuwaiq** |
| Total Organic Carbon Content (wt %) | Maximum | 4.98 | 3.9 | 13.17 |
| | Minimum | 1.00 | 0.15 | |
| | Average | 2.44 | 1.19 | 4.9 |
| S1 Pyrolysis Yield (mg HC / g rock) | Maximum | 4.69 | 4.58 | 12.04 |
| | Minimum | 0.32 | 0.02 | 0.39 |
| | Average | 1.05 | 0.77 | 3.4 |
| S2 Pyrolysis Yield (mg HC / | Maximum | 7.55 | 6.18 | 18.8 |
| | Minimum | 0.55 | 0.02 | 0.54 |
| g rock) | Average | 2.53 | 0.8 | 3.4 |
| Hydrogen Index (S2*100/TOC) | Maximum | 231 | 218 | 194 |
| | Minimum | 63 | 50 | 48 |
| | Average | 112 | 102 | 91 |
| Tmax (°C) | Maximum | 476 | 479 | 493 |
| | Minimum | 434 | 456 | 455 |
| | Average | 462 | 471 | 475 |

As shown in Table 1, the source rock formations Tuwaiq and Jubaila possess high source rock quality, whereas source rock formation Hanifa possesses low potential source facies. Thus, the subsequent experiments performed and described herein focused on the source rock formations Tuwaiq and Jubaila as primary and secondary targets for shale gas exploration.

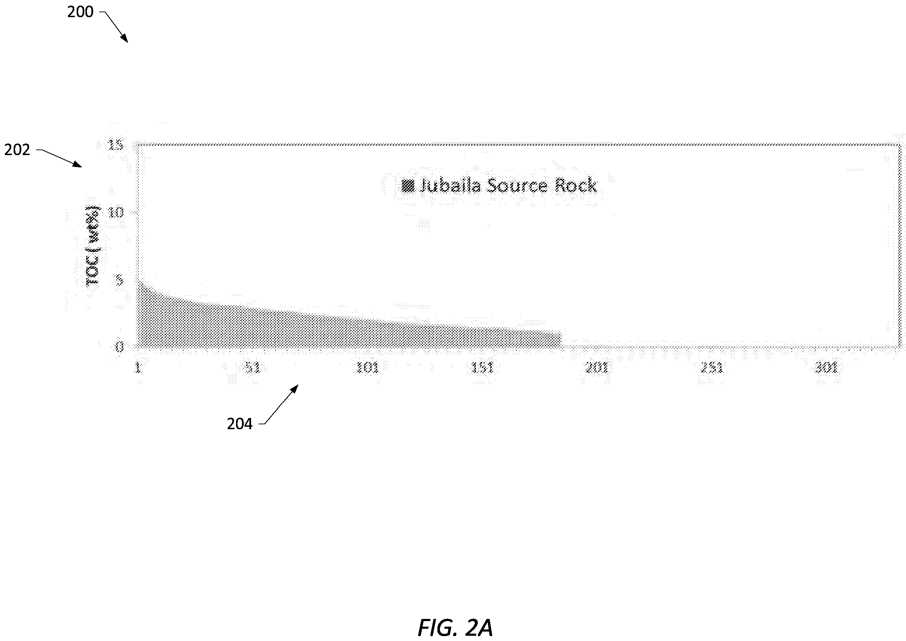

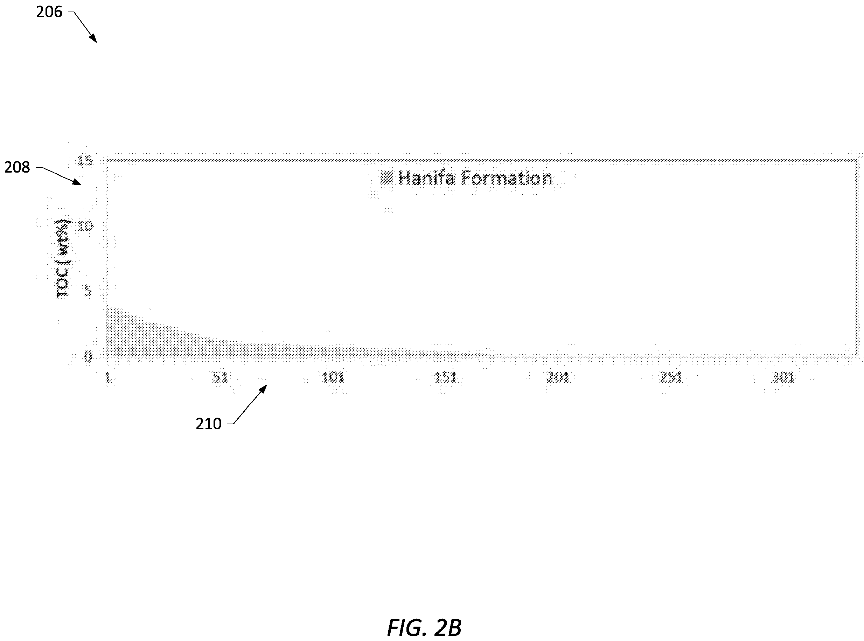

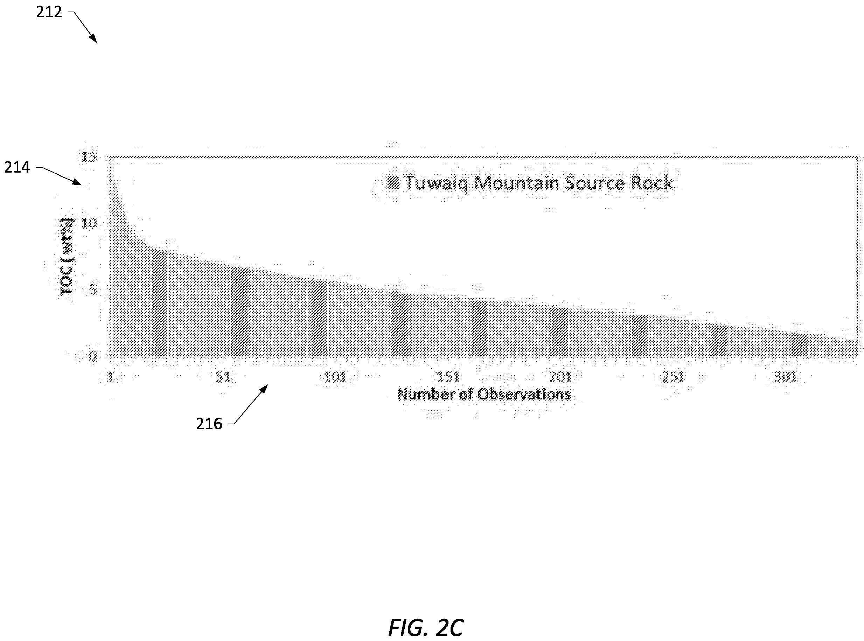

FIGS. 2A-2C are graphs that depict the distribution of total organic carbon (TOC) for the three source rock formations Tuwaiq, Hanifa, and Jubaila. FIG. 2A depicts a graph 200 of TOC wt% (on the y-axis 202) vs. depth (on the x-axis 204) for source rock formation Jubaila. FIG. 2B depicts a graph 206 of TOC wt% (on the y-axis 208) vs. depth (on the x-axis 210) for source rock formation Hanifa. FIG. 2C depicts a graph 112 of TOC wt% (on the y-axis 214) vs. depth (on the x-axis 216) for source rock formation Tuwaiq.

As shown in FIG. 2C, the graph 212 for the Tuwaiq source rock formation illustrates cyclic total organic carbon (TOC) distributions most likely due to fluctuations in relative sea level. The average TOC values of 4.9% and maximum TOC values of 13.17% confirm the potential of the Tuwaiq source rock for production. At present depth and present day thermal maturity, the Tuwaiq source rock has hydrogen indices of 48 mg HC/g TOC to 194 mg HC/g TOC that suggests a type II and type III source rock. As shown in the stratigraphic cross section shown in FIG. 1, the thickness of the Tuwaiq rock formation is relatively uniform between the exploration wells with a slight increase in thickness noted toward the west at Well-A.

In addition to the core sampling, MGIL sampling was performed on each on the five exploration wells: Well-A, Well-B, Well-C, Well-D, and Well-E to enable capture of representative gases from the source rock formations. The MGIL sampling collected one sample about every 150 feet (45.72m) (ft) in the nonproductive zones to establish a background gas trend. Once the background was established, the MGIL sampling interval was decreased to about 500 ft (152.4m) or greater on subsequent wells. During gas shows, the sampling interval was increased to about one sample every 30 ft (9.144m) of penetration. The isotopic ratio of the different gas components were analyzed using an isotope ratio mass spectrometer (IRMS).

The accuracy of the pre-drilling seismic estimated tops and formation thicknesses was compared to the actual stratigraphic depths encountered during drilling of the exploration wells to determine the number of representative gas samples obtained. As the Hanifa rock was found to be a predominantly non-source facies, a minimum number of samples were selected for isotopic analysis for this source rock. For thicker intervals, the top, middle, and bottom-third portions of the source rock were sampled. In some instances, such as the Jubaila rock in exploration wells Well-A and Well-B, the source interval (excluding poor or non-source facies) was found to be non-existent or insufficiently resolved. The isotopic data not present in Table 2 was the result of non-selection or, in some instances such as deuterium analysis, a gas component concentration below the analytical minimum. Table 2 depicts carbon-13 isotopic data in methane (δ¹³C₁), ethane (δ¹³C₂), propane (δ¹³C₃), i-butane (δ¹³iC₄), n-butane (δ¹³nC₄), i-pentane (δ¹³iC₅), and n-pentane (δ¹³iC₅) for the three source rock formations Hanifa, Source T, and Jubaila and the five exploratory wells, with the isotopic data expressed as delta (δ) deviation of a sample relative to the Pee Dee Belemnite (PDB) reference standard. Table 3 depicts deuterium isotopic data in methane (δDC₁), ethane (δDC₂), propane (δDC₃) for the three source rock formations Hanifa, Source T, and Jubaila and the five exploratory wells, with the isotope data expresses as delta (δ) deviation of sample relative to the PDB reference standard:

**Table 2: Carbon isotopic data for Hanifa, Source T, and Jubaila formations and Well-A, Well-B, Well-C, Well-D, and Well-E exploratory wells**

| **Well** | **Sample No.** | **Source Rock Formation** | **δ¹³C₁** | **δ¹³C₂** | **δ¹³C₃** | **δ¹³iC₄** | **δ^{B}nC₄** | **δ¹³iC₅** | **δ¹³nC₅** |
|---|---|---|---|---|---|---|---|---|---|
| Well-A | 1 | Hanifa | -59.6 | -41.2 | -30.9 | -30.3 | -27.8 | -28.4 | -28.4 |
| Well-A | 2 | Tuwaiq | -59.7 | -41.1 | -30.6 | -30.0 | -27.7 | -28.7 | -28.3 |
| Well-A | 3 | Tuwaiq | -60.1 | -40.5 | -30.4 | -30.6 | -27.6 | -28.9 | -27.9 |
| Well-B | 1 | Jubaila | -56.1 | -31.1 | -25.8 | -29.5 | -25.1 | -27.3 | -25.9 |
| Well-B | 2 | Hanifa | -56.3 | -33.6 | -27.8 | -30.7 | -26.9 | -28.2 | -25.9 |
| Well-B | 3 | Hanifa | -56.1 | -32.1 | -26.7 | -30.5 | -25.6 | -27.2 | -25.1 |
| Well-B | 4 | Tuwaiq | -56.0 | -31.3 | -25.6 | -29.8 | -24.9 | -26.9 | -24.2 |
| Well-C | 1 | Jubaila | -52.8 | -33.7 | -28.0 | -30.1 | -26.9 | -29.2 | -33.4 |
| Well-C | 2 | Jubaila | -53.2 | -33.5 | -28.0 | | | | |
| Well-C | 3 | Jubaila | -56.2 | -33.2 | -27.8 | | -26.8 | -28.4 | -32.8 |
| Well-C | 4 | Hanifa | -56.2 | -31.7 | -26.6 | -30.5 | -26.1 | -28.6 | -34.5 |
| Well-C | 5 | Hanifa | -56.4 | -31.8 | -26.8 | -31.7 | -26.7 | -28.1 | -34.3 |
| Well-C | 6 | Tuwaiq | -55.9 | -30.5 | -25.5 | | | | |
| Well-C | 7 | Tuwaiq | -55.5 | -30.5 | -25.5 | -29.4 | -24.7 | -27.2 | -30.1 |
| Well-C | 8 | Tuwaiq | -56.0 | -30.7 | -25.4 | -30.1 | -24.7 | -27.0 | -31.4 |
| Well-C | 9 | Tuwaiq | -55.7 | -30.5 | -25.3 | | | | |
| Well-D | 1 | Jubaila | -50.2 | -29.7 | -26.8 | -29.7 | -26.1 | -28.7 | -25.2 |
| Well-D | 2 | Jubaila | -50.4 | -30 | -26.3 | -29.4 | -25.9 | -27.7 | -24.8 |
| Well-D | 3 | Jubaila | -50.2 | -30 | -26.3 | -28.9 | -25.6 | -27.3 | -24.1 |
| Well-D | 4 | Hanifa | -52.4 | -30.2 | -26.1 | -29.4 | -25.2 | -27.1 | -24.1 |
| Well-D | 5 | Tuwaiq | -53.8 | -29.8 | -25.9 | -29.8 | -24.8 | -27.1 | -23.9 |
| Well-D | 6 | Tuwaiq | -53.8 | -29.4 | -25.5 | -29.4 | -24.4 | -26.5 | -23.8 |
| Well-D | 7 | Tuwaiq | -54.1 | -29.4 | -25.1 | -29.4 | -24.2 | -26.6 | -23.9 |
| Well-D | 8 | Tuwaiq | -53.7 | -30 | -25.9 | -30 | -24.6 | -26.6 | -24 |
| Well-E | 1 | Jubaila | -50.5 | -30.5 | -27.8 | -31.0 | -26.2 | -29.3 | -25.2 |
| Well-E | 2 | Jubaila | -50.9 | -29.6 | -26.3 | -30.1 | -25.2 | -28.5 | -24.8 |
| Well-E | 3 | Jubaila | -51.0 | -29.1 | -25.3 | -29.2 | -24.1 | -27.7 | -23.7 |
| Well-E | 4 | Hanifa | -56.6 | -28.6 | -24.6 | -30.4 | -22.8 | -26.9 | -22.0 |
| Well-E | 5 | Hanifa | -56.3 | -28.2 | -22.5 | -30.2 | -22.5 | -26.7 | -23.0 |
| Well-E | 6 | Hanifa | -53.0 | -26.5 | -22.7 | -28.8 | -22.0 | -25.9 | -22.6 |
| Well-E | 7 | Tuwaiq | -51.3 | -25.4 | -22.1 | -28.3 | -21.8 | -25.5 | -21.8 |
| Well-E | 8 | Tuwaiq | -51.5 | -25.3 | -22.1 | -28.3 | -21.5 | -25.3 | -22.1 |
| Well-E | 9 | Tuwaiq | -50.7 | -24.4 | -20.8 | | | | |

**Table 3: Deuterium isotopic data for Hanifa, Source T, and Jubaila formations and Well-A, Well-B, Well-C, Well-D, and Well-E exploratory wells**

| **Well** | **Sample No.** | **Source Rock Formation** | **δDC₁** | **δDC₂** | **δDC₃** |
|---|---|---|---|---|---|
| Well-A | 1 | Hanifa | -329 | -148 | -99 |
| Well-A | 2 | Tuwaiq | -336 | -149 | -103 |
| Well-A | 3 | Tuwaiq | -335 | -147 | -101 |
| Well-B | 1 | Jubaila | -272 | | |
| Well-B | 2 | Hanifa | -292 | -118 | -97 |
| Well-B | 3 | Hanifa | | | |
| Well-B | 4 | Tuwaiq | -292 | -118 | -96 |
| Well-C | 1 | Jubaila | -265 | | |
| Well-C | 2 | Jubaila | -265 | | |
| Well-C | 3 | Jubaila | -266 | | |
| Well-C | 4 | Hanifa | -290 | -100 | |
| Well-C | 5 | Hanifa | | | |
| Well-C | 6 | Tuwaiq | -310 | -116 | |
| Well-C | 7 | Tuwaiq | -293 | -105 | -91 |
| Well-C | 8 | Tuwaiq | -294 | -113 | |
| Well-C | 9 | Tuwaiq | -306 | -118 | |
| Well-D | 1 | Jubaila | -224 | -128.0 | -105.0 |
| Well-D | 2 | Jubaila | -220 | -125.0 | -103.0 |
| Well-D | 3 | Jubaila | -223 | -130.0 | -105.0 |
| Well-D | 4 | Hanifa | -256 | -120.0 | -103.0 |
| Well-D | 5 | Tuwaiq | -278 | -110.0 | -96.0 |
| Well-D | 6 | Tuwaiq | -281 | -107.0 | -98.0 |
| Well-D | 7 | Tuwaiq | -281 | -109.0 | -97.0 |
| Well-D | 8 | Tuwaiq | -279 | -109.0 | -93.0 |
| Well-E | 1 | Jubaila | -225 | | |
| Well-E | 2 | Jubaila | -233 | | |
| Well-E | 3 | Jubaila | -229 | | |
| Well-E | 4 | Hanifa | -267 | | |
| Well-E | 5 | Hanifa | -273 | | |
| Well-E | 6 | Hanifa | -280 | | |
| Well-E | 7 | Tuwaiq | -277 | | |
| Well-E | 8 | Tuwaiq | -269 | | |
| Well-E | 9 | Tuwaiq | -270 | | |

As will be appreciated, thermal maturity may be used to evaluate the generative potential of source rock. One technique for estimating the thermal maturity of source rock is vitrinite reflectance (%%Ro). As used herein, the term "vitrinite" refers to a type of maceral that originates from the woody tissue of vascular plants. In some instances where vitrinite is unavailable or rare, such as carbonate depositional environments, the reflectance of solid bitumen or other vitrinite-like macerals may be used to estimate thermal maturity. In such instances, the resulting measurement of the reflectance of solid bitumen or other vitrinite-like macerals may be converted to a normalized vitrinite reflectance equivalent (VRo-eq). Alternatively, organic matter type and maturity may be determined using Rock Eval® or other pyrolysis techniques. For the exploration wells described in this example, present day thermal maturity was measured and calculated for all samples using Rock Eval® and solid bitumen optical reflectance techniques. Techniques such Rock Eval® or the determination of reflectance of vitrinite, bitumen, or other macerals requires the obtaining of core samples from the targeted formation.

As discussed above, pyrolysis analysis techniques such as Rock Eval® may provide a determination of the temperature (Tmax) at which the maximum rate of hydrocarbon generation occurs in a kerogen sample. As shown in Table 1, the average Tmax value for Tuwaiq source rock is 475°C, with Tmax values as low as 455°C in Well A and as high as 493°C in Well E. These Tmax values suggest that the Tuwaiq source rock thermal maturity ranges between a late oil to early dry gas thermal maturity window. VRo-eq was estimated from Tmax data using the known Jarvie's conversion formula, as shown below in Equation 1: $VRo - eq = 0.018 \times Tmax - 7.16$ (1)

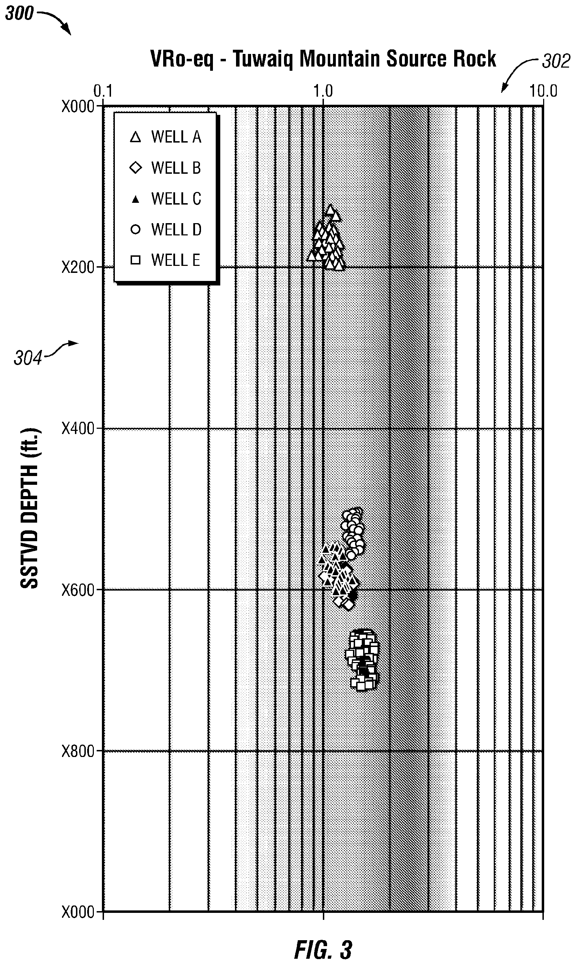

Where VRo-eq is the vitrinite reflectance equivalent and Tmax is the temperature at which the maximum rate of hydrocarbon generation occurs in a kerogen sample during pyrolysis analysis. The VRo-eq calculated for Tuwaiq source rock using Equation 1 ranged from 1.0% in Well-A to 1.55% in Well-E with an average VRo-eq of 1.39%. FIG. 3 is a plot 300 of the vitrinite reflectance equivalent determined by Equation 1 (on the x-axis 302) vs. depth (on the y-axis 304) for the Tuwaiq source rock. As shown in FIG. 3, a clear increase of VRo-eq with depth was observed.

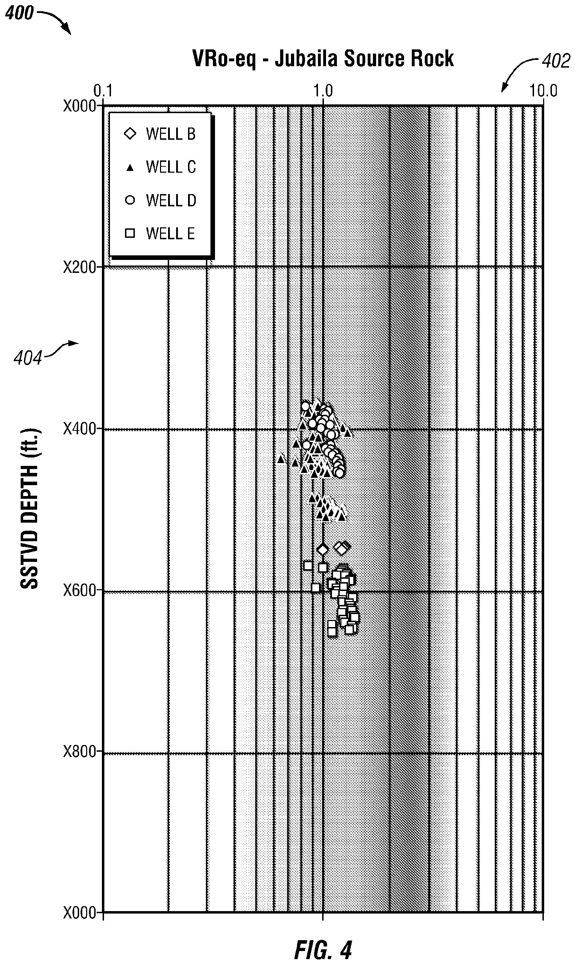

As shown in Table 1, the Jubaila source rock recorded an average TOC of 2.44% with a maximum of 4.98%. The hydrogen indices of organic matter in the formation rock are between 63 mg HC/g TOC and 231 mg HC/g TOC. As also shown in Table 1, the average Tmax value recorded for the Jubaila source rock was 462°C with a Tmax as low as 453°C for Well-A corresponding to an oil generation maturity window. A maximum average Tmax of 469°C was recorded for the Jubaila source rock was recorded in Well-E, corresponding to a wet gas generation maturity window. The VRo-Eq calculated from Tmax according to Equation 1 ranged from 0.99% at Well-A to 1.28% at Well-E with an average VRo-eq of 1.1%. FIG. 4 is a plot 400 of the vitrinite reflectance equivalent (on the x-axis 402) determined by Equation 1 vs. depth (on the y-axis 404) for Well-B, Well-C, Well-D, and Well-E for the Jubaila source rock.

To confirm the VRo-eq determinations using the Jarvie conversion formula shown in Equation 1, the thermal maturity was also determined from solid bitumen (BitRo) fragments from Tuwaiq core samples. VRo-eq was estimated from solid bitumen reflectance (BitRo) using the known Jacob's conversion formula, as shown below in Equation 2: $VRo - eq = 0.618 \times BitRo - 0.4$ (2)

Where VRo-eq is the vitrinite reflectance equivalent and BitRo is the solid bitumen reflectance.

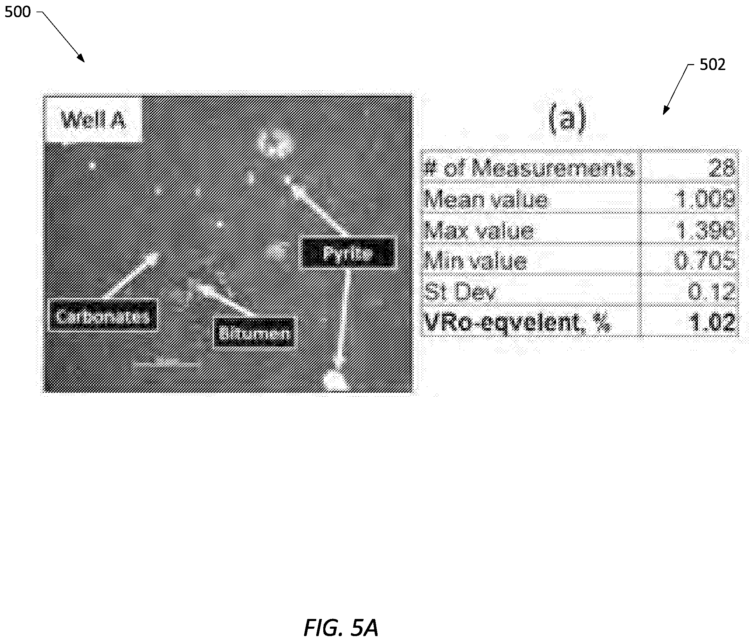

FIGS. 5A-5E depict bitumen reflectance data and images of representative core samples from Tuwaiq source rock for the five exploratory wells Well-A through Well-E. The measured samples were generally rick in organic matter content and included solid bitumen in various forms co-deposited with carbonate grains and, in some instances, associated with farmboidal pyrite. A network of amorphous bituminite and traces of inertinite was also observed in the sample matrix. For Well-E a small amount of organic particles corresponding to indigenous vitrinite were also encountered.

FIG. 5A depicts an image 500 of a representative core sample obtained from Well-A and a table 502 with the corresponding bitumen reflectance (BitRo) and VRo-eq values. As shown in FIG. 5A, the mean BitRo for a Tuwaiq source rock core sample at Well-A was 1.01% based on 28 readings, which resulted in a VRo-eq of 1.02% determined using Equation 2. Thus, the VRo-eq value calculated from BitRo is in agreement with the VRo-eq determined from Tmax using Equation 1. Both VRo-eq determination techniques suggest that the Tuwaiq source rock organic matter in Well-A is presently at a thermal maturity corresponding to the oil window of hydrocarbon generation.

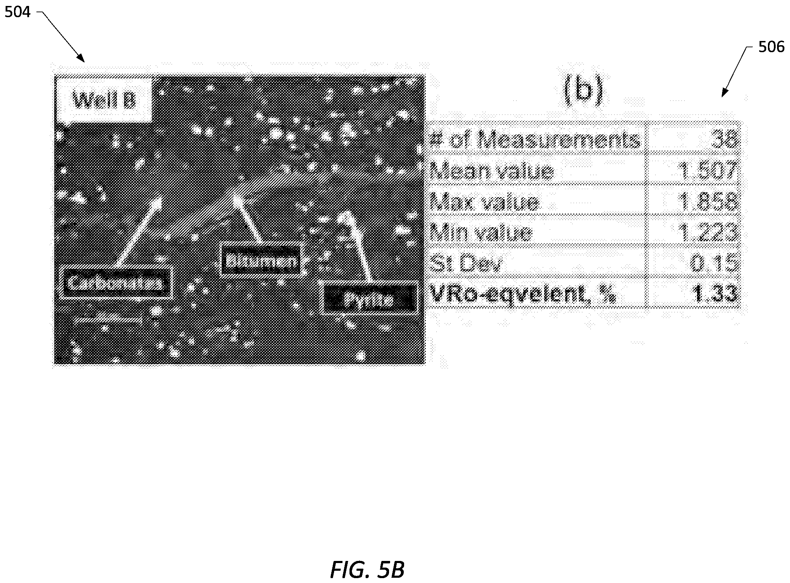

FIG. 5B depicts an image 504 of a representative core sample obtained from Well-B and a table 506 with the corresponding bitumen reflectance (BitRo) and VRo-eq values. As shown in FIG. 5B, the mean BitRo for a Tuwaiq source rock core sample at Well-B was 1.5% based on 38 readings, which resulted in a VRo-Eq of 1.33% determined using Equation 2. Here again, the VRo-Eq value calculated from BitRo is in agreement with the VRo-Eq determined from Tmax using Equation 1. Both VRo-Eq determination techniques suggest that the Tuwaiq source rock organic matter in Well-B is presently at the wet gas window of hydrocarbon generation.

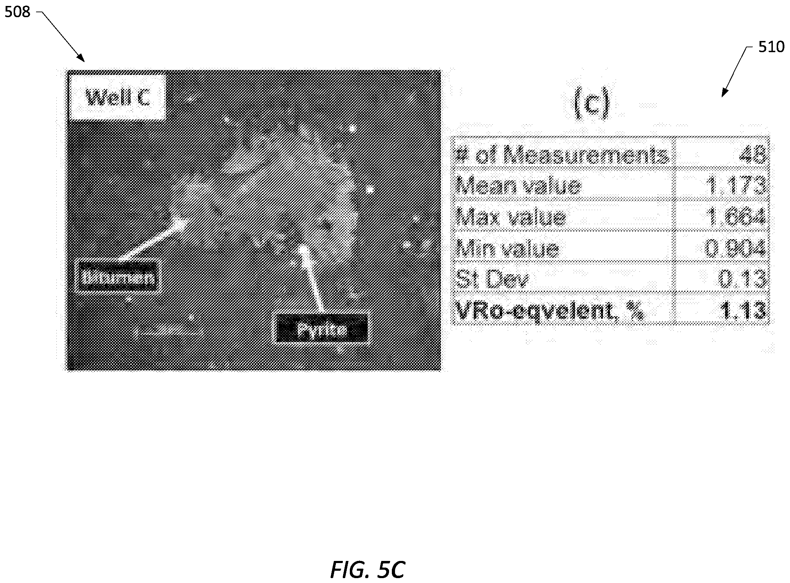

FIG. 5C depicts an image 508 of a representative core sample obtained from Well-C and a table 510 with the corresponding bitumen reflectance (BitRo) and VRo-eq value. As shown in FIG. 5C, the mean BitRo for a Tuwaiq source rock core sample at Well-C was 1.2% based on 48 readings, which resulted in a VRo-Eq of 1.13% determined using Equation 2. The VRo-Eq value calculated from BitRo is again comparable to the VRo-Eq determined from Tmax using Equation 1. Both VRo-Eq determination techniques suggest that the Tuwaiq source rock organic matter in Well-C is presently at the late oil window of hydrocarbon generation.

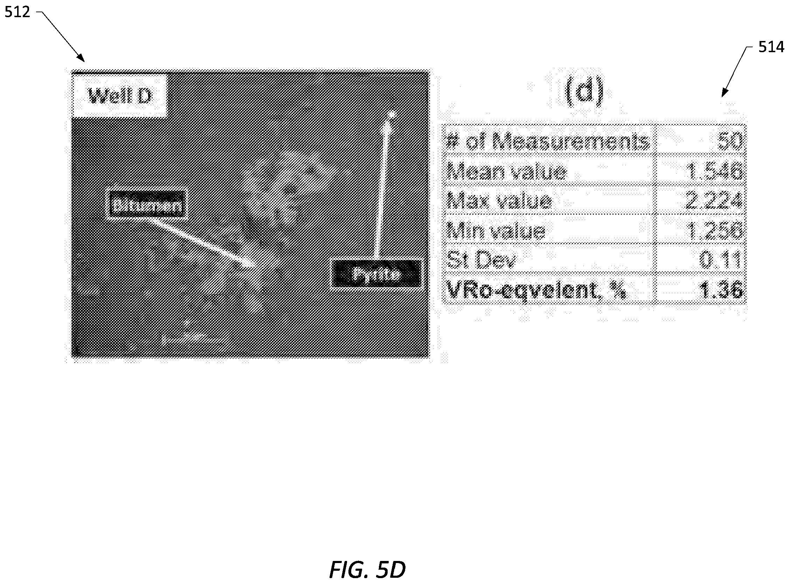

FIG. 5D depicts an image 512 of a representative core sample obtained from Well-D and a table 514 with the corresponding bitumen reflectance (BitRo) and VRo-eq value. As shown in FIG. 5D, the mean BitRo for a Tuwaiq source rock core sample at Well-D was 1.54% based on 50 readings, which resulted in a VRo-Eq of 1.36% determined using Equation 2. For Well-D, the VRo-Eq value calculated from BitRo is again comparable to the VRo-Eq determined from Tmax using Equation 1. Both VRo-Eq determination techniques suggest that the Tuwaiq source rock organic matter in Well-D is presently at the late wet gas window of hydrocarbon generation.

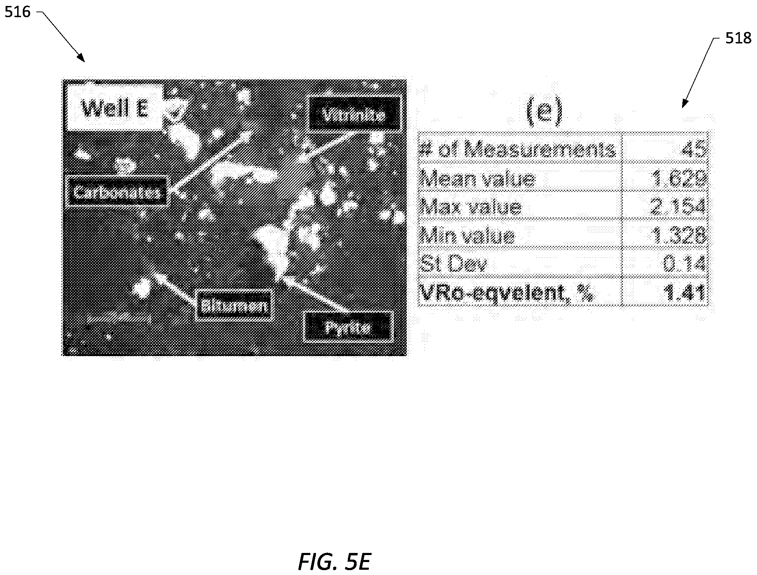

FIG. 5E depicts an image 516 of a representative core sample obtained from Well-E and a table 518 with the corresponding bitumen reflectance (BitRo) and VRo-eq value. As shown in FIG. 5E, the mean BitRo for a Tuwaiq source rock core sample at Well-E was 1.63% based on 45 readings, which resulted in a VRo-Eq of 1.36% determined using Equation 2. The VRo-Eq determined from Tmax using Equation 1 was about 1.55% different than the VRo-Eq determined from the BitRo value. As such, one of the VRo-Eq estimation techniques may be less sensitive at slightly higher thermal maturities. As mentioned above, however, organic particles of indigenous vitrinite were obtained in Well-E. Using these particles, the mean %Ro of the primary vitrinite was determined to be 1.5% based on 6 measurements. Thus, the %Ro value determined from the primary vitrinite shows a better correlation with the VRo-Eq determined from Tmax (1.55%) than the VRo-Eq determined from BitRo (1.36%). The thermal maturity measured from primary vitrinite and determined from Tmax according to Equation 1 indicate that the Tuwaiq source rock organic matter in Well-E is very mature and at the dry gas window of hydrocarbon generation.

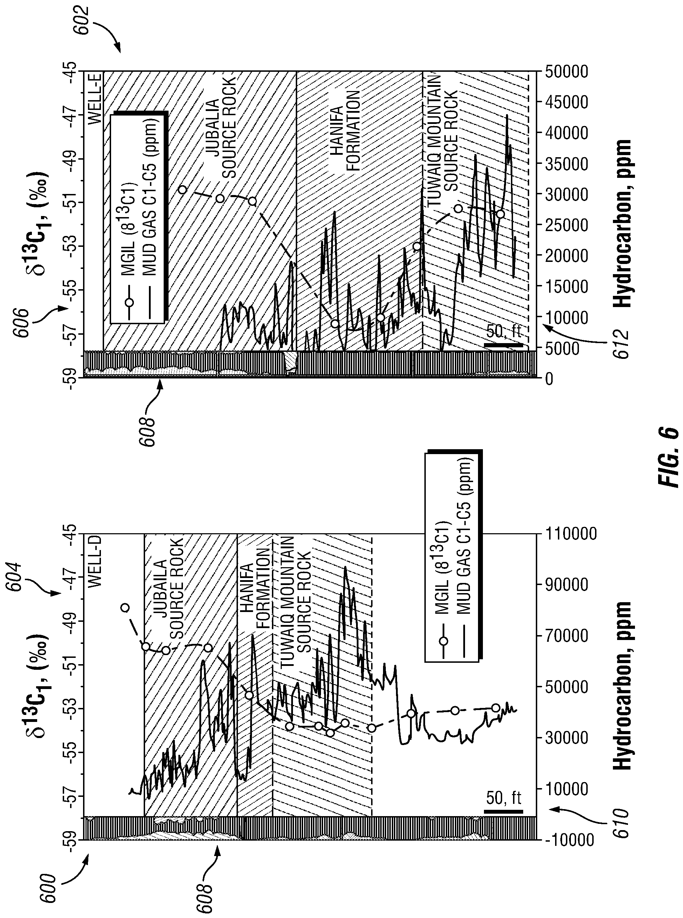

FIG. 6 depicts side-by-side depth plots for exploration wells Well-D and Well-E and illustrates general geological data with gas sampling locations. The plot 600 depicts geological data for Well-D and the plot 602 depicts geological data for Well-E. Each plot 600 and 602 depicts the measured methane carbon isotopic values (δ¹³C₁ on the upper x-axes 604 and 606) vs depth (on the y-axes 608) and the recorded well site drilling mud gas (C1-C5 total gas) measurements (on the lower x-axes 610 and 612) vs. depth. The y-axes 608 depict depths of 550 ft from the same starting depth and encompassing all three source rock targets Jubaila, Hanifa, and Tuwaiq.

FIG. 6 illustrates the depth variations between the two wells across more than 80 kilometers (km) of the basin, for which the depth differential in Tuwaiq is only about 170 ft and suggestive of a wide shallow geological basin. The mud gas measurements on the x-axes 610 and 612 illustrate, for each of Jubaila and Tuwaiq source rock, stronger gas shows towards the basal sections. This data is typical of transgressive depositional cycles where the richest organic matter and the higher derived gas shows may be found within each formation.

As noted above, the upper x-axes 604 and 606 depicted in FIG. 6 show the measured methane carbon isotopic values for each gas sampling location, and the plots 600 and 602 depict the interpolated trend of methane carbon isotopic values. In general, isotopic trends with increasing depth generally result in less negative isotopic values due to progressive generation of isotopically heaver methane gas via thermal cracking of indigenous kerogen. However, as shown in FIG. 6, for Well-D the shallower Jubaila methane carbon isotopic values are significantly less negative than the deeper Tuwaiq source rock, suggesting a distinct difference between the Jubaila and Tuwaiq source organic matter. As also shown in the plot 602 in FIG. 6, for Well-E there were no observed significant differences between the Jubaila and Tuwaiq source rock formations. However, the Tuwaiq methane gas isotopic values for Well-E have increased over those from Well-D, likely as a result of the higher geothermal gradient observed in the vicinity of Well-E.

Using the Tuwaiq source rock data from Table 1 and the gas isotope data from Table 2 from nearly equivalent depth intervals, accurate thermal maturity relationships were established between the thermal maturity (as indicated by VRo-Eq) and the gas isotope data. Table 3 depicts the thermal maturity calibration data taken from Tables 1 and 2. As discussed above, the VRo-Eq from Tmax was calculated according to Equation 1, and the VRo-Eq from bitumen reflectance was calculated according to Equation 2. As also discussed above, samples having primary vitrinite were obtained from Well-E and used to directly measure vitrinite reflectance using optical measurements. The fifth column of Table 4 depicts the directly measured vitrinite reflectance:

**Table 4: Vitrinite reflectance equivalents, vitrinite reflectance, and isotopic values**

| **Well** | **Tmax** | **VRo-Eq from Tmax** | **VRo-Eq from solid bitumen** | **Vitrinite Reflectance (%Ro)** | **δ¹³C₁** | **δDC₁** |
|---|---|---|---|---|---|---|
| Well A | 455 | 1.03 | 1.02 | NA | -59.7 | -336 |
| Well C | 465 | 1.21 | 1.14 | NA | -55.8 | -301 |
| Well B | 472 | 1.34 | 1.33 | NA | -56.3 | -292 |
| Well D | 477 | 1.43 | 1.36 | NA | -53.9 | -280 |
| Well E | 484 | 1.55 | 1.40 | 1.5 | -51.4 | -273 |

As shown in Table 4, the comparison between the three vitrinite reflectance (i.e., Tmax-based vitrinite reflectance equivalence, bitumen-based vitrinite reflectance equivalence, and directly measured vitrinite reflectance) values indicates a high confidence in the thermal maturity determinations. The relationship between the vitrinite reflectance (as an indicator of thermal maturity) and the measured gas isotopic values of entrained generated hydrocarbon gases may be used to develop a thermal maturity determination based on isotopic data.

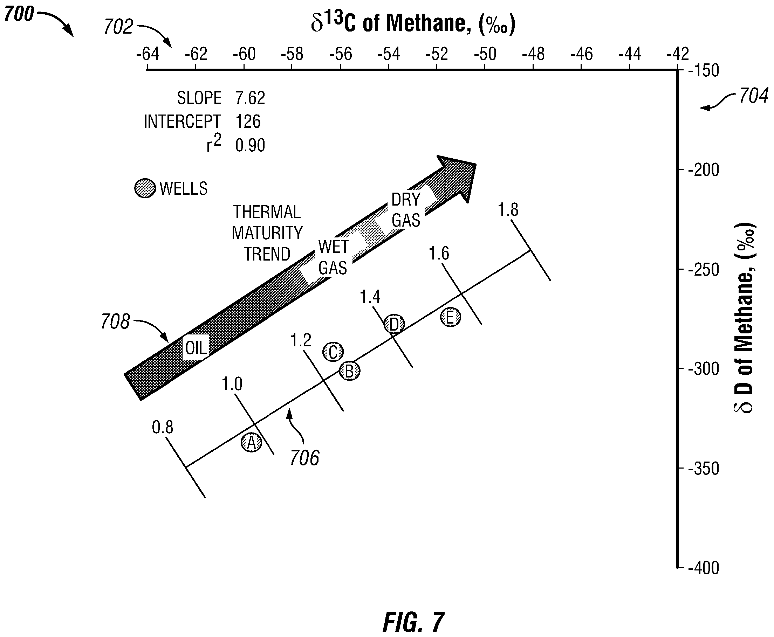

FIG. 7 is a plot 700 illustrating the relationship between methane carbon-13 (δ¹³C₁) and deuterium (δDC₁) isotopic data, and vitrinite reflectance based on the values from Table 3. The plot 700 depicts methane carbon-13 (on the x-axis 702) and deuterium (on the y-axis 704), with the vitrinite reflectance values for each of Well-A, Well-B, Well-C, Well-D, and Well-E plotted along the line 706. As shown by arrow 708, the increasing vitrinite reflectance values correspond to an increasing thermal maturity trend (e.g., from oil to wet gas to dry gas).

In some embodiments, a least squared regression analysis may be applied to the data in Table 3 to determine the relationship between the vitrinite reflectance (as an indicator of thermal maturity) and the measured gas isotopic values of entrained generated hydrocarbon gases. In some embodiments, a relationship between the vitrinite reflectance and methane carbon-13 isotope may be determined. For example, for the Tuwaiq source rock, the least squares regression analysis yielded a correlation coefficient (r2) value of 0.9 for the relationship between the vitrinite reflectance equivalent and methane carbon-13 isotope, such that the relationship may be defined according to Equation 3: $VRo - Eq = 0.069 \times {δ}^{13} {C}_{1} + 5.13$ (3)

Where VRo-Eq is the vitrinite reflectance equivalent and 5¹³C₁ is the isotopic value of methane carbon-13 as determined from MGIL data. A similar relationship between vitrinite reflectance equivalent and methane deuterium may also be determined using a least squares regression analysis or other suitable analysis.

The gas isotopic thermal maturity model depicted in Equation 3 may be used to determine vitrinite reflectance equivalence (and, thus, thermal maturity) in source rock and identify "sweet spots" for drilling. For example, the determination of thermal maturity from isotopic values may be developed and used in locations where no or few reliable source rock samples are available, or in situations (for example, tight gas plays) where only captured hydrocarbon gases are feasible for extraction. Thus, the thermal maturity determined from gas isotopic values may be used to evaluate thermal maturities and the production potential of development wells in such locations.

### PROCESSES AND SYSTEMS FOR THERMAL MATURITY DETERMINATIONS

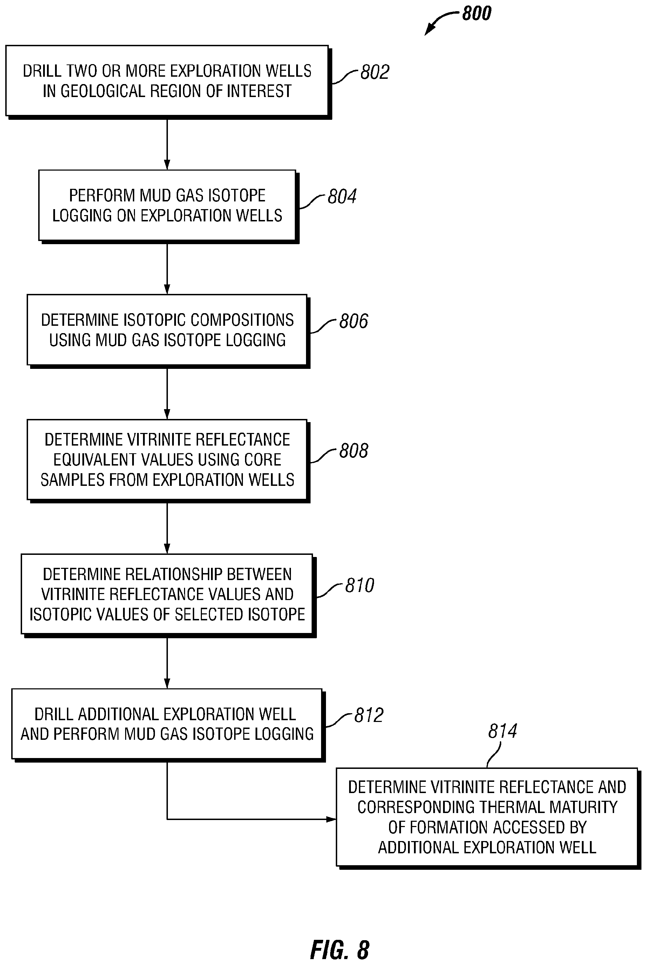

FIG. 8 depicts a process 800 for determining thermal maturity from gas isotopic values obtained from mud gas isotope logging (MGIL) in accordance with an embodiment of the disclosure. Initially, two or more exploration wells may be drilled into a geological region of interest (block 802). For example, the exploration wells may traverse multiple source rock formations at different depths in the geological region of interest.

During drilling of the wells, mud gas isotope logging may be performed on the exploration wells (block 804). The isotopic values in mud gas may be determined using mud gas isotope logging (block 806), as discussed above. For example, in some embodiments, the isotopic values may include carbon-13 and deuterium isotopic values in mud gases such as methane, ethane, propane, i-butane, n-butane, i-pentane, and n-pentane.

Next, vitrinite reflectance equivalent (VRo-Eq) values may be determined using core samples from the exploration wells using known techniques. In accordance with the invention, vitrinite reflectance equivalent values are determined from the temperature (Tmax) at which the maximum rate of hydrocarbon generation occurs in a kerogen sample during pyrolysis analysis, as discussed above and shown in Equation 1. Additionally or alternatively, vitrinite reflectance equivalent values are determined using bitumen reflectance, as discussed above and shown in Equation 2.

Next, a relationship between vitrinite reflectance equivalent (and thermal maturity) and the isotopic values for a selected isotope may be determined (block 810). For example, the selected isotope may be carbon-13 and the isotopic values may be methane carbon-13. In other embodiments, other isotopes and isotopic values may be used. In some embodiments, the selected isotope may be deuterium and the isotopic values may be methane deuterium, ethane deuterium, or propane deuterium. In some embodiments, the selected isotope may be carbon-13 and the isotopic values may be ethane carbon-13, propane carbon-13, i-butane carbon-13, n-butane carbon-13, i-pentane carbon-13, or n-pentane carbon-13.

The vitrinite reflectance equivalent (as an indicator of thermal maturity) may then be determined from isotopic values subsequently obtained from mud gas isotope logging data. For example, as shown in FIG. 8, another exploration well may be drilled and mud gas isotope logging may be performed on the well (block 812). Using the mud gas isotope logging data, the vitrine reflectance and the corresponding thermal maturity of the well may be determined (block 814). In some embodiments, the vitrinite reflectance equivalent may be compared to a range of vitrinite reflectance values that correspond to different thermal maturities. For example, a vitrinite reflectance value in the range of about 0.8 to about 1.3 may indicate a thermal maturity of oil, a vitrinite reflectance value in the range of greater than about 1.3 to about 1.5 may indicate a thermal maturity of wet gas, and a vitrinite reflectance value in the range of greater than about 1.5 may indicate a thermal maturity of dry gas.

The vitrinite reflectance and corresponding thermal maturity may thus be determined from isotopic values without obtaining a core sample or performing other time-consuming and costly procedures that require additional trips downhole. As discussed above, the mud gas isotope logging may be performed on return mud during drilling the additional exploration, thus enabling near real-time determination of vitrinite reflectance and corresponding thermal maturity for formations encountered at various depths during drilling. In some embodiments, lateral drilling operations may use mud gas isotope logging and the determination of vitrinite reflectance and corresponding thermal maturity to assess the effectiveness of hydraulic fracturing treatments in a formation and to ensure maximum in-zone completions to optimize production potential.

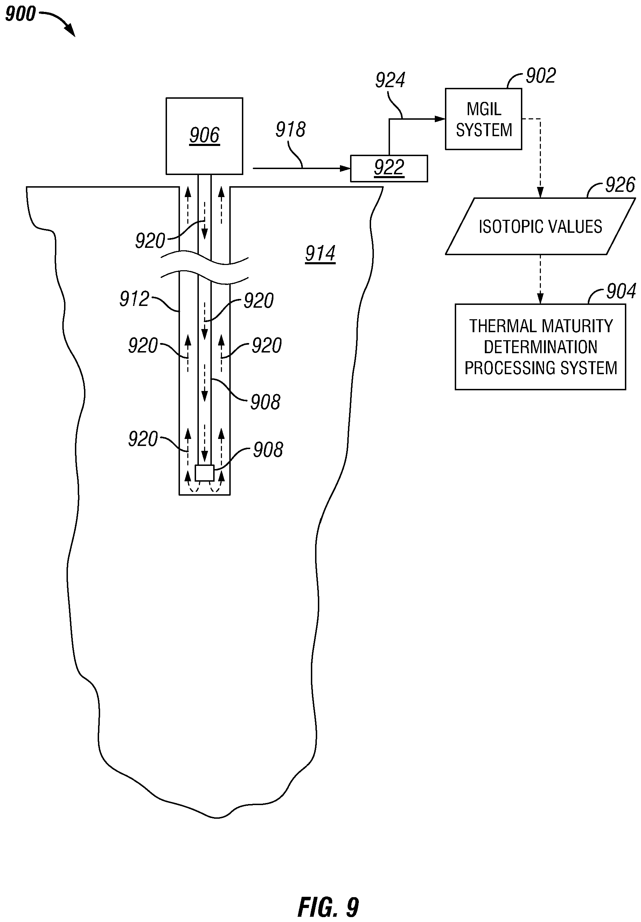

FIG. 9 depicts a well site 900 having a mud gas isotope logging (MGIL) system 902 and a thermal maturity determination processing system 904 in accordance with an embodiment of the disclosure. The drilling environment includes a drilling system 906 configured to operate a drill 908 connected to a drill pipe 910 extending into a wellbore 912 (also referred to as a "borehole") formed in a shale formation 914. Although FIG. 9 depicts the wellbore 912 and the drilling system 906 in a generally vertical orientation, it should be appreciated that in other embodiments other orientations may be used.

The wellbore 912 may include any form of a hole formed in a geologic formation. In some embodiments, the wellbore 912 may include a wellbore created for the purpose of locating and extracting hydrocarbons or other resources from the formation 916. For example, the formation 916 may include an oil and gas reservoir, and the wellbore 912 may include a wellbore drilled into the formation 916 for the purpose of locating and obtaining information about the oil, gas and other hydrocarbons in the reservoir.

As will be appreciated, a drilling mud (also referred to as a "drilling fluid") may be circulated around the drill 908 via the drill pipe 910 and the wellbore 912, as shown by arrows 918, to lubricate the drill 908 and remove cuttings generated by drilling into the formation 916. The drilling mud may return to the surface, as shown by arrow 920, for collection and processing, as shown by block 922. The collection and processing of the return drilling mud may include various components that remove solids such as cuttings from the return mud. The collection and processing may include also include a degasser that removes gases 924 from the return mud. The gas removed from the return mud may be collected or sampled via the mud gas isotope logging system 902. The mud gas isotopic logging system 902 may analyze the gases 924 removed from the drilling mud and determine the isotopic values 926 of the gases. For example, the mud gas isotopic logging system 902 may include an isotope ratio mass spectrometer (IRMS) for analyzing mud gases removed from the drilling mud.

The isotopic values 926 are in accordance with the present invention processed by a thermal maturity determination processing system 904 to determine an isotopic maturity model in accordance with embodiments of the disclosure. The thermal maturity determination processing system 904 in accordance with the invention, includes processor, a memory, and optionally a display. The processor (as used the disclosure, the term "processor" encompasses microprocessors) may include one or more processors having the capability to receive and process data received from the mud gas isotopic logging system 902. The processor may also include single-core processors and multicore processors and may include graphics processors.

The thermal maturity determination processing system 904 also includes a memory (which may include one or more tangible non-transitory computer readable storage mediums) that may include volatile memory, such as random access memory (RAM), and non-volatile memory, such as ROM, flash memory, a hard drive, any other suitable optical, magnetic, or solid-state storage medium, or a combination thereof. The memory of the thermal maturity determination processing system 904 stores executable computer code that includes executable computer code for processing data received from the mud gas isotopic logging system 902 and displaying data received from the mud gas isotopic logging system 902, such as on a display of the thermal maturity determination processing system 904.

Accordingly, the mud gas isotopic logging system 902 may also include a display, such as a liquid crystal display (LCD), an organic light emitting diode (OLED) display, or other suitable display. The display may display a user interface (for example, a graphical user interface) that may enable a user to obtain and view data from the thermal maturity determination processing system 904. In some embodiments, the thermal maturity determination processing system 904 may include a touch screen display and may include or be provided with touch sensitive elements through which a user may interact with the user interface.

In some embodiments, the thermal maturity determination processing system 904 may include a network interface that provides for communication between the thermal maturity determination processing system 904 and other computers remote from the well site 900. Such a network interface may include a wired network interface card (NIC), a wireless (e.g., radio frequency) network interface card, or combination thereof. In some embodiments, the thermal maturity determination processing system 904 may provide for the transmission of vitrinite reflectance relationships and determinations to another computer remote from a well site.

The invention is defined by appended claims. Further modifications and alternative embodiments of various aspects of the disclosure will be apparent to those skilled in the art in view of this description. Accordingly, this description is to be construed as illustrative only and is for the purpose of teaching those skilled in the art the general manner of carrying out the embodiments described herein. It is to be understood that the forms shown and described herein are to be taken as examples of embodiments. used herein are for organizational purposes only and are not meant to be used to limit the scope of the description.

## Claims

1. A method for determining the thermal maturity of a rock formation in a geological region of interest for hydrocarbon production, comprising:
drilling a plurality of exploration wells to access the rock formation;
obtaining mud gas isotope logging data for each of the plurality of exploration wells, the mud gas isotope logging data comprising isotopic values associated with an isotope in a mud gas;
obtaining a vitrinite reflectance equivalent for each of the plurality of exploration wells, the vitrinite reflectance equivalent determined from a core sample (500, 504, 508, 512, 516) from each of the plurality of exploration wells; and
determining a relationship between vitrinite reflectance equivalent and the isotopic values, the vitrinite reflectance equivalent indicating a thermal maturity of the rock formation, wherein obtaining the vitrinite reflectance equivalent for each of the plurality of exploration wells comprises:
determining, for each of the plurality of exploration wells,
a temperature at which the maximum rate of hydrocarbon generation occurs in a kerogen sample from the respective exploration well during a pyrolysis analysis; and/or
an amount of bitumen in a core sample (500, 504, 508, 512, 516) obtained from the respective exploration well; and
calculating the vitrinite reflectance equivalent from the temperature and/or the bitumen amount for each of the plurality of exploration wells.

2. The method of claim 1, wherein the rock formation is a first rock formation, comprising:
determining the vitrinite reflectance equivalent using the relationship and isotopic values associated with an additional exploration well accessing a second rock formation; and
identifying a thermal maturity of the second rock formation using the vitrinite reflectance equivalent.

3. The method of any one of the preceding claims, comprising identifying a drilling location in the geological region of interest for hydrocarbon production using the thermal maturity indicated by the vitrinite reflectance equivalent.

4. The method of any one of the preceding claims, wherein the mud gas comprises methane and the isotope comprises carbon-13.

5. The method of any one of the preceding claims, wherein the mud gas comprises hydrogen and the isotope comprises deuterium.

6. The method of any one of the preceding claims, wherein determining a relationship between the vitrinite reflectance equivalent and the isotopic values comprises performing a least squares regression analysis on a set of vitrinite reflectance equivalent values and a set of isotopic values.

7. A system adapted to determine the thermal maturity of a rock formation in a geological region of interest for hydrocarbon production, the system comprising:
a mud gas isotope logging system (902) configured to receive mud gas and determine isotopic values associated with an isotope in the mud gas;
a thermal maturity determination processing system (904) comprising a processor and a non-transitory computer-readable memory accessible by the processor, the memory having executable code stored thereon, the executable code comprising a set of instructions that causes the processor to perform operations comprising:
receiving, from the mud gas isotope logging system (902), mud gas isotope logging data for each of a plurality of exploration wells, the mud gas isotope logging data comprising isotopic values associated with the isotope in the mud gas;
obtaining a vitrinite reflectance equivalent for each of the plurality of exploration wells, the vitrinite reflectance equivalent determined from a core sample (500, 504, 508, 512, 516) from each of the plurality of exploration wells; and
determining a relationship between vitrinite reflectance equivalent and the isotopic values, the vitrinite reflectance equivalent indicating a thermal maturity of the rock formation, wherein obtaining the vitrinite reflectance equivalent for each of the plurality of exploration wells comprises:
determining, for each of the plurality of exploration wells,
a temperature at which the maximum rate of hydrocarbon generation occurs in a kerogen sample from the respective exploration well during a pyrolysis analysis; and/or
an amount of bitumen in a core sample (500, 504, 508, 512, 516) obtained from the respective exploration well;
and calculating the vitrinite reflectance equivalent from the temperature and/or bitumen amount for each of the plurality of exploration wells.

8. The system of claim 7, the operations comprising:
determining a vitrinite reflectance equivalent associated with an additional exploration well using the relationship and an isotopic value associated with the additional exploration well; and
identifying a thermal maturity of the rock formation using the vitrinite reflectance equivalent.

9. The system of claims 7 or 8, wherein the mud gas comprises methane and the isotope comprises carbon-13.

10. The system of claims 7, 8, or 9, wherein the mud gas comprises hydrogen and the isotope comprises deuterium.

11. The system of claims 7, 8, 9 or 10 wherein determining a relationship between vitrinite reflectance equivalent and the isotopic values comprises performing a least squares regression analysis on a set of vitrinite reflectance equivalent values and a set of isotopic values.

## Patentansprüche

1. Verfahren zum Bestimmen der thermischen Reife einer Gesteinsformation in einer für die Kohlenwasserstoff-Förderung relevanten Region, Folgendes umfassend:
Bohren einer Vielzahl von Erkundungsbohrungen, um Zugang zur Gesteinsformation zu erhalten;
Erzielen von Schlammgas-Isotop-Loggingdaten für jede der Vielzahl von Erkundungsbohrungen, wobei die Schlammgas-Isotop-Loggingdaten Isotopenwerte umfassen, welche einem Isotop in einem Schlammgas zugeordnet sind;
Erzielen eines Vitrinitreflexionsgleichwertes für jede der Vielzahl von Erkundungsbohrungen, wobei der Vitrinitreflexionsgleichwert anhand einer Kernprobe (500, 504, 508, 512, 516) von jeder der Vielzahl von Erkundungsbohrungen bestimmt wird; und
Bestimmen einer Beziehung zwischen dem Vitrinitreflexionsgleichwert und den Isotopenwerten, wobei der Vitrinitreflexionsgleichwert eine thermische Reife der Gesteinsformation angibt, wobei Erzielen des Vitrinitreflexionsgleichwertes für jede der Vielzahl von Erkundungsbohrungen Folgendes umfasst:
Bestimmen, für jede der Vielzahl von Erkundungsbohrungen,
einer Temperatur, bei welcher die maximale Rate an Kohlenwasserstofferzeugung in einer Kerogenprobe aus der jeweiligen Erkundungsbohrung im Zuge einer pyrolytischen Analyse eintritt; und/oder
einer Menge an Teer in einer aus der jeweiligen Erkundungsbohrung erzielten Kernprobe (500, 504, 508, 512, 516); und
Berechnen des Vitrinitreflexionsgleichwertes anhand der Temperatur und/oder der Teermenge für jede der Vielzahl von Erkundungsbohrungen.

2. Verfahren nach Anspruch 1, wobei die Gesteinsformation eine erste Gesteinsformation ist, Folgendes umfassend:
Bestimmen des Vitrinitreflexionsgleichwertes unter Verwendung der Beziehung und der Isotopenwerte, welche einer zusätzlichen Erkundungsbohrung zugeordnet sind, welche Zugang zu einer zweiten Gesteinsformation gibt; und
Identifizieren einer thermischen Reife der zweiten Gesteinsformation unter Verwendung des Vitrinitreflexionsgleichwertes.

3. Verfahren nach einem der vorhergehenden Ansprüche, umfassend Identifizieren einer Bohrposition in der für die Kohlenwasserstoff-Förderung relevanten geologischen Region, unter Verwendung der durch den Vitrinitreflexionsgleichwert angegebenen thermischen Reife.

4. Verfahren nach einem der vorhergehenden Ansprüche, wobei das Schlammgas Methan umfasst und das Isotop Kohlenstoff 13 umfasst.

5. Verfahren nach einem der vorhergehenden Ansprüche, wobei das Schlammgas Wasserstoff umfasst und das Isotop Deuterium umfasst.

6. Verfahren nach einem der vorhergehenden Ansprüche, wobei Bestimmen einer Beziehung zwischen dem Vitrinitreflexionsgleichwert und den Isotopenwerten Durchführen einer Regressionsanalyse nach der Methode der kleinsten Quadrate an einem Satz von Vitrinitreflexionsgleichwerten und an einem Satz von Isotopenwerten umfasst.

7. System, welches zum Bestimmen der thermischen Reife einer Gesteinsformation in einer für die Kohlenwasserstoff-Förderung relevanten Region geeignet ist, wobei das System Folgendes umfasst:
ein Schlammgas-Loggingsystem (902), welches konfiguriert ist, um Schlammgas zu empfangen und Isotopenwerte zu bestimmen, welche einem Isotop in dem Schlammgas zugeordnet sind;
ein Verarbeitungssystem (904) zur Bestimmung einer thermischen Reife, welches einen Prozessor und einen nicht transienten, computerlesbaren Speicher umfasst, auf welchen der Prozessor zugreifen kann, wobei der Speicher darin gespeicherten ausführbaren Code aufweist, wobei der ausführbare Code einen Satz Befehle umfasst, welcher den Prozessor veranlasst, folgende Schritte durchzuführen:
Empfangen, von dem Schlammgas-Isotop-Loggingsystem (902), von Schlammgas-Isotop-Loggingdaten für jede einer Vielzahl von Erkundungsbohrungen, wobei die Schlammgas-Isotop-Loggingdaten Isotopenwerte umfassen, welche dem Isotop in dem Schlammgas zugeordnet sind;
Erzielen eines Vitrinitreflexionsgleichwertes für jede der Vielzahl von Erkundungsbohrungen, wobei der Vitrinitreflexionsgleichwert anhand einer Kernprobe (500, 504, 508, 512, 516) von jeder der Vielzahl von Erkundungsbohrungen bestimmt wird; und
Bestimmen einer Beziehung zwischen dem Vitrinitreflexionsgleichwert und den Isotopenwerten, wobei der Vitrinitreflexionsgleichwert eine thermische Reife der Gesteinsformation angibt, wobei Erzielen des Vitrinitreflexionsgleichwertes für jede der Vielzahl von Erkundungsbohrungen Folgendes umfasst:
Bestimmen, für jede der Vielzahl von Erkundungsbohrungen,
einer Temperatur, bei welcher die maximale Rate an Kohlenwasserstofferzeugung in einer Kerogenprobe aus der jeweiligen Erkundungsbohrung im Zuge einer pyrolytischen Analyse eintritt; und/oder
einer Menge an Teer in einer aus der jeweiligen Erkundungsbohrung erzielten Kernprobe (500, 504, 508, 512, 516);
und Berechnen des Vitrinitreflexionsgleichwertes anhand der Temperatur und/oder der Teermenge für jede der Vielzahl von Erkundungsbohrungen.

8. System nach Anspruch 7, wobei die Schritte Folgendes umfassen:
Bestimmen eines einer zusätzlichen Erkundungsbohrung zugeordneten Vitrinitreflexionsgleichwertes unter Verwendung der Beziehung und eines der zusätzlichen Erkundungsbohrung zugeordneten Isotopenwertes; und
Identifizieren einer thermischen Reife der Gesteinsformation unter Verwendung des Vitrinitreflexionsgleichwertes.

9. System nach einem der Ansprüche 7 oder 8, wobei das Schlammgas Methan umfasst und das Isotop Kohlenstoff 13 umfasst.

10. System nach einem der Ansprüche 7, 8 oder 9, wobei das Schlammgas Wasserstoff umfasst und das Isotop Deuterium umfasst.

11. System nach einem der Ansprüche 7, 8, 9 oder 10, wobei Bestimmen einer Beziehung zwischen dem Vitrinitreflexionsgleichwert und den Isotopenwerten Durchführen einer Regressionsanalyse nach der Methode der kleinsten Quadrate an einem Satz von Vitrinitreflexionsgleichwerten und an einem Satz von Isotopenwerten umfasst.

## Revendications

1. Procédé pour la détermination de la maturité thermique d'une formation rocheuse dans une région géologique d'intérêt pour une production d'hydrocarbures, comprenant :
le forage d'une pluralité de puits d'exploration pour accéder à la formation rocheuse;
l'obtention de données de diagraphie d'isotopes de gaz de boues pour chacun de la pluralité de puits d'exploration, les données de diagraphie d'isotopes de gaz de boues comprenant des valeurs isotopiques associées à un isotope dans un gaz de boues;
l'obtention d'une équivalence de réflectance de la vitrinite pour chacun de la pluralité de puits d'exploration, l'équivalence de réflectance de la vitrinite déterminée à partir d'un échantillon de carotte (500, 504, 508, 512, 516) à partir de chacun de la pluralité de puits d'exploration; et
la détermination d'une relation entre l'équivalence de réflectance de la vitrinite et les valeurs isotopiques, l'équivalence de réflectance de la vitrinite indiquant une maturité thermique de la formation rocheuse, dans laquelle l'obtention de l'équivalence de réflectance de la vitrinite pour chacun de la pluralité de puits d'exploration comprend :
la détermination, pour chacun de la pluralité de puits d'exploration,
d'une température à laquelle le taux maximal de production d'hydrocarbures est atteint dans un échantillon de kérogène à partir du puits d'exploration respectif lors d'une analyse par pyrolyse; et/ou
une quantité de bitume dans un échantillon de carotte (500, 504, 508, 512, 516) obtenu à partir du puits d'exploration respectif; et
le calcul de l'équivalence de réflectance de la vitrinite à partir de la température et/ou de la quantité de bitume pour chacun de la pluralité de puits d'exploration.

2. Procédé selon la revendication 1, dans lequel la formation rocheuse est une première formation rocheuse, comprenant :
la détermination de l'équivalence de réflectance de la vitrinite en utilisant la relation et des valeurs isotopiques associées à un puits d'exploration additionnel accédant à une seconde formation rocheuse; et
l'identification d'une maturité thermique de la seconde formation rocheuse en utilisant l'équivalence de réflectance de la vitrinite.

3. Procédé selon l'une quelconque des revendications précédentes, comprenant l'identification d'un emplacement de forage dans la région géologique d'intérêt pour la production d'hydrocarbures en utilisant la maturité thermique indiquée par l'équivalence de réflectance de la vitrinite.

4. Procédé selon l'une quelconque des revendications précédentes, dans lequel le gaz de boues comprend du méthane et l'isotope comprend du carbone 13.

5. Procédé selon l'une quelconque des revendications précédentes, dans lequel le gaz de boues comprend de l'hydrogène et l'isotope comprend du deutérium.

6. Procédé selon l'une quelconque des revendications précédentes, dans lequel la détermination d'une relation entre l'équivalence de réflectance de la vitrinite et les valeurs isotopiques comprend la réalisation d'une analyse de régression des moindres carrés sur un ensemble de valeurs d'équivalence de réflectance de la vitrinite et un ensemble de valeurs isotopiques.

7. Système adapté pour déterminer la maturité thermique d'une formation rocheuse dans une région géologique d'intérêt pour la production d'hydrocarbures, le système comprenant :
un système de diagraphie d'isotopes de gaz de boues (902) configuré pour recevoir du gaz de boues et déterminer des valeurs isotopiques associées à un isotope dans le gaz de boues;
un système de traitement (904) de détermination de maturité thermique comprenant un processeur et une mémoire lisible par ordinateur non transitoire accessible par le processeur, la mémoire ayant un code exécutable stocké dessus, le code exécutable comprenant un ensemble d'instructions qui amènent le processeur à effectuer des opérations comprenant :
la réception, du système de diagraphie d'isotopes de gaz de boues (902), de données de diagraphie d'isotopes de gaz de boues pour chacun de la pluralité de puits d'exploration, les données de diagraphie d'isotopes de gaz de boues comprenant des valeurs isotopiques associées à l'isotope dans le gaz de boues;
l'obtention d'une équivalence de réflectance de la vitrinite pour chacun de la pluralité de puits d'exploration, l'équivalence de réflectance de la vitrinite étant déterminée à partir d'un échantillon de carotte (500, 504, 508, 512, 516) à partir de chacun de la pluralité de puits d'exploration; et
la détermination d'une relation entre une équivalence de réflectance de la vitrinite et les valeurs isotopiques; l'équivalence de réflectance de la vitrinite indiquant une maturité thermique de la formation rocheuse, dans laquelle l'obtention de l'équivalence de réflectance de la vitrinite pour chacun de la pluralité de puits d'exploration comprend :
la détermination, pour chacun de la pluralité de puits d'exploration,
d'une température à laquelle le taux maximal de production d'hydrocarbures est atteint dans un échantillon de kérogène à partir du puits d'exploration respectif lors d'une analyse par pyrolyse; et/ou
une quantité de bitume dans un échantillon de carotte (500, 504, 508, 512, 516) obtenu à partir du puits d'exploration respectif;
et le calcul de l'équivalence de réflectance de la vitrinite à partir de la température et/ou de la quantité de bitume pour chacun de la pluralité de puits d'exploration.

8. Système selon la revendication 7, les opérations comprenant :
la détermination d'une équivalence de réflectance de la vitrinite associée à un puits d'exploration additionnel en utilisant la relation et une valeur isotopique associée au puits d'exploration additionnel; et
l'identification d'une maturité thermique de la formation rocheuse en utilisant l'équivalence de réflectance de la vitrinite.

9. Système selon les revendications 7 ou 8, dans lequel le gaz de boues comprend du méthane et l'isotope comprend du carbone 13.

10. Système selon les revendications 7, 8, ou 9, dans lequel le gaz de boues comprend de l'hydrogène et l'isotope comprend du deutérium.

11. Système selon les revendications 7, 8, 9 ou 10, dans lequel la détermination d'une relation entre une équivalence de réflectance de la vitrinite et les valeurs isotopiques comprend la réalisation d'une analyse de régression des moindres carrés sur un ensemble de valeurs d'équivalence de réflectance de la vitrinite et un ensemble de valeurs isotopiques.
